Europäisches Patentamt

European Patent Office

Office européen des brevets

(11) Veröffentlichungsnummer: **0 246 515 B1**

# EUROPÄISCHE PATENTSCHRIFT

(45) Veröffentlichungstag der Patentschrift: **19.11.92**

(21) Anmeldenummer: **87106716.1**

(22) Anmeldetag: **08.05.87**

(51) Int. Cl.⁵: **B06B 3/02**, B05B 17/06

(54) **Ultraschall-MHz-Schwinger, insbesondere zur Flüssigkeitszerstäubung.**

(30) Priorität: **20.05.86 DE 3616713**

(43) Veröffentlichungstag der Anmeldung:
**25.11.87 Patentblatt 87/48**

(45) Bekanntmachung des Hinweises auf die
Patenterteilung:
**19.11.92 Patentblatt 92/47**

(84) Benannte Vertragsstaaten:
**AT BE CH DE ES FR GB IT LI NL SE**

(56) Entgegenhaltungen:
**DE-A- 1 425 897       DE-A- 2 557 958
US-A- 3 904 896       US-A- 4 301 968
US-A- 4 474 326       US-A- 4 541 564**

**THE JOURNAL OF THE ACOUSTICAL SOCIE-
TY OF AMERICA, Band 51, Nr. 3, Teil 2, 1972,
Seiten 953-959, New York, US; A. BARONE et
al.: "Flexural vibrating free-edge plates with
stepped thicknesses for generating high directional ultrasonic radiation"**

(73) Patentinhaber: **SIEMENS AKTIENGESELL-
SCHAFT
Wittelsbacherplatz 2
W-8000 München 2(DE)**

(72) Erfinder: **Drews, Wolf-Dietrich, Dr. Dipl.-Phys.
Breslauer Strasse 6
W-8620 Lichtenfels(DE)**
Erfinder: **Van der Linden, Klaus
Innerer Ring 40
W-8640 Kronach(DE)**

## Beschreibung

Die Erfindung bezieht sich auf einen Ultraschall-MHz-Schwinger mit einer Anregungsenergie ≤ 30 W (Watt) zur Zerstäubung von kleinsten Flüssigkeitsmengen zur Erzeugung eines lungengängigen Aerosols.

Aus der US-A-3 904 896 und US-A-3 738 574 ist ein piezoelektrisches Schwingsystem, insbesondere für Flüssigkeitszerstäubung, bekannt mit einem anregenden Schwinger aus einer Biegeschwingerplatte mit Wandlerplatte, wobei die Biegeschwingerplatte mittels eines Steges mit einer dünneren Arbeitsplatte gekoppelt ist. Die Biegeschwingerplatte und der Steg können auch ineinander übergehend als Kegelstumpf ausgebildet sein und zusammen mit der Arbeitsplatte ein Stück bilden. Solche Schwingersysteme können zur Zerstäubung von Flüssigkeiten in kHz-Ultraschallzerstäubern eingesetzt werden. Ein Nachteil der bekannten kHz-Ultraschallzerstäuber ist es, daß die Durchmesser der erzeugten Tröpfchen wesentlich größer sind als für die Erzeugung eines lungengängigen Aerosols erforderlich ist. Damit ist auch meist weniger als 50 % der erzeugten Aerosolmasse nicht lungengängig. Lungengängiges Aerosol erhält man hier nur mit zusätzlichen mechanischen Abscheidungssystemen für die größeren Tröpfchen.

Ein auch für medizinische Zwecke einsetzbarer Ultraschallzerstäuber für Flüssigkeiten ist durch die DE-A-14 25 897 bekannt geworden. Bei diesem Ultraschallzerstäuber ist die abstrahlende Fläche je nach Länge des Ultraschallzerstäubers konkav oder konvex ausgebildet. Auch ist eine schalenförmige Vertiefung zur Aufnahme der zu zerstäubenden Flüssigkeiten offenbart. In dieser Druckschrift wird darauf hingewiesen, daß eine schalenförmige Vertiefung in der Abstrahlfläche für die Flüssigkeit den Nachteil hat, daß diese je nach Dicke der Flüssigkeitstiefe eine unterschiedliche Bedämpfung bedeutet, die eine optimale Ausnutzung der Ultraschallenergie im Wege steht. (vgl. Seite 4, 2. Absatz, erster Teil).

Durch die US-Patentschrift US-A-3 301 968 ist es bei einem im kHz-Bereich schwingenden Ultraschallzerstäuber für Brennstoffe bekannt die Abstrahlfläche konkav zu wölben und für den Anschluß einer Flüssigkeitszufuhr mit einer Anschlußbohrung zu versehen. Mit der Wölbung der Abstrahlfläche soll hier der Kegel, in dem die Tröpfchen versprüht werden, erweitert werden.

Durch die US-Patentschrift US-A-4 474 326 ist es bei einem anderen im kHz-Bereich schwingenden Ultraschallzerstäuber für den Einsatz an Ölbrennern mit einem sich verjüngenden metallischen Amplitudentransformator mit Piezokeramikscheibe und mit einer die Abstrahlfläche bildenden plattenförmigen Verbreiterung bekannt, die Abstrahlfläche zur Halterung der zu zerstäubenden Flüssigkeit mit einer kleinen Vertiefung oder eine Riffelung zu versehen. Dadurch wird die Gefahr des seitlichen Abtropfens von Flüssigkeit vermieden.

Bekannt ist auch die Zerstäubung mittels eines im MHz-Bereich schwingenden Kalottenschwingers. Dieser benötigt jedoch eine Gegenmasse und arbeitet über eine Vorlaufwassersäule und hat daher einen im Verhältnis zur Menge des erzeugten Aerosols recht hohen Leistungsbedarf. Mit ihm wird ein Aerosol von unterschiedlichem Durchmesser, nämlich von ca. 0,1 $\mu$m bis zu mehreren Millimetern, erhalten. Mittels eines mechanischen Tröpfchenabscheiders müssen die Tröpfchen größeren Durchmessers zurückgehalten werden.

Aufgabe der Erfindung ist es, kleinste Flüssigkeitsvolumina (V ≤ 15 $\mu$l) im MHz-Bereich (MHz < f ≤ 5 MHz) ohne ein mechanisches Tröpfchenfilter bei geringster elektrischer Anregungsleistung (P ≤ 30 W) und ohne Ankopplung über Flüssigkeitsmedien in Tröpfchen < 40 $\mu$m zu zerstäuben. Eine auf einmal auf einen Ultraschallzerstäuber dosierte Flüssigkeitsmenge soll innerhalb kürzester Zeit lageunabhängig zerstäubt werden können.

Diese Aufgabe wird durch die im Patentanspruch 1 angegebenen Merkmale gelöst. Weitere Ausgestaltungen und Weiterbildungen der Erfindung gehen aus den Unteransprüchen hervor.

Dadurch, daß der Ultraschall-MHz-Schwinger mit einem sich im wesentlichen verjüngenden metallischen Amplitudentransformator, mit einer an seiner breiteren Basisfläche angekoppelten Piezokeramikscheibe und mit einem an der hierzu gegenüberliegenden verjüngten Seite des Amplitudentransformators, die zu einem Hals ausgebildet ist, befindlichen plattenförmigen Verbreiterung, die zur Halterung der Flüssigkeit bei Schräglagen mit durchgehend konkaver, hinsichtlich Durchmesser und Wölbung ganz an die zu zerstäubende Flüssigkeitsmenge und Flüssigkeitsoberflächenspannung angepaßter Oberfläche ohne Flüssigkeitszuführungsbohrung versehen ist, und dadurch, daß die Höhe des Amplitudentransformators zuzüglich der Dicke der plattenförmigen Verbreiterung ein Vielfaches, vorzugsweise das Vier- bis Sechsfache, einer halben Wellenlänge des Ultraschalls im MHz-Bereich im Amplitudentransformator entspricht, ist die Voraussetzung für die Lösung der gestellten Aufgabe geschaffen worden.

Die an dem oberen Ende des Amplitudentransformators befestigte Verbreiterung nimmt in ihrem Hohlraum die zu zerstäubende Flüssigkeit auf. Die Verbreiterung ermöglicht es, die zugeführte Flüssigkeitsmenge an den für die Zerstäubung optimalen Punkt zu bringen. Die Wölbung der Verbreiterung ist auf die Flüssigkeitsmenge und die Oberflächenspannung der Flüssigkeit abgestimmt, die durch ihre Eigenschaften und Formgebung der Kavitationskeimbildung dienlich sind. Die Abmessungen der Verbreiterung sind so gewählt, daß die zu zerstäubende Flüssigkeitsmenge gut aufgenommen wird und auch bei verschiedenen Schräglagen des Ultraschallzerstäubers nicht abtropft.

Die gewählte Geometrie erlaubt auch bei Vergießen mit Silikonkautschuk einen gleich guten effizienten Betrieb.

Bei Ultraschallzerstäubern gemäß der Erfindung arbeitet die Piezokeramik - im Gegensatz zu den bekannten im MHz-Bereich in Dickenrichtung arbeitenden Ultraschallzerstäubern - ohne Gegenmasse und ohne Ankopplung an ein Flüssigkeitsvolumen. Es werden nahezu ausschließlich die für die Inhalationstherapie benötigten kleinen Tröpfchendurchmesser erzeugt. Eine mechanische Abscheidung der großen Tropfen wird nicht benötigt. Diese mechanische Tröpfchenselektierung ist bei den zur Zeit bekannten MHz-Zerstäubern erforderlich.

Mit der Erfindung ist es möglich, kleinste Flüssigkeitsvolumina, $v \leq 15 \ \mu l$, im Frequenzbereich von 1 MHz $\leq f \leq$ 5 MHz, mit geringster elektrischer Anregungsleistung (P $\leq$ 30 W) zu zerstäuben, wobei 50 % des Flüssigkeitsvolumens in Tröpfchen < 40 $\mu m$ umgewandelt werden. Die überwiegende Zahl der Tröpfchen hat einen Durchmesser < 5 $\mu m$. Ein mechanisches Tröpfchenfilter ist daher nicht erforderlich. Die Erfindung ermöglicht es, eine auf einmal in die hohlspiegelartige Verbreiterung dosierte Flüssigkeitsmenge ohne Ankopplung über Flüssigkeitsmedien innerhalb kürzester Zeit, das heißt lageunabhängig zu zerstäuben.

Als Piezokeramik sind die bekannten piezoelektrischen Materialien geeignet, insbesondere Blei-Zirkonat-Titanat.

Das Material des Amplitudentransformators besteht vorzugsweise aus kavitations- und korrosionsbeständigem CrNi-Stahl. Die Verbreiterung besteht vorzugsweise aus dem Material des Amplitudentransformators oder aber auch aus mit Metallpulver gefüllten Kunststoffen.

Die Gesamthöhe des Amplitudentransformators, bestehend aus der Höhe des eigentlichen Amplitudentransformators zuzüglich der Höhe des Randes der Verbreiterung, soll ein Vielfaches, vorzugsweise das Vier- bis Sechsfache, der halben Wellenlänge im Amplitudentransformator sein.

Die Dicke der Verbreiterung entspricht bevorzugt einer halben Wellenlänge in Ultraschall, kann jedoch von 1/4 bis 1,5 Wellenlängen betragen.

Der Durchmesser der Verbreiterung soll vorzugsweise ein Mehrfaches, vorzugsweise das Drei- bis Siebenfache, des Durchmessers des Halses betragen.

Der Durchmesser des Halses beträgt vorzugsweise das Ein- bis Dreifache der Wellenlänge des Ultraschalls im Amplitudentransformator.

Schwinger gemäß der Erfindung können allgemein zum Zerstäuben von Flüssigkeiten eingesetzt werden. Mit besonderem Vorteil werden sie zum Zerstäuben von Medikamenten und Duftstoffen, wäßrigen Lösungen oder auch Wasser verwendet. Besonders empfehlenswert ist die Zerstäubung, wenn Tröpfchengrößen bis maximal 50 $\mu m$ benötigt werden, die lungengängig sein sollen. Bei Verwendung in Inhalatoren ist der netzunabhängige Akkubetrieb hervorzuheben und auch die mögliche kleine Bauform der Geräte.

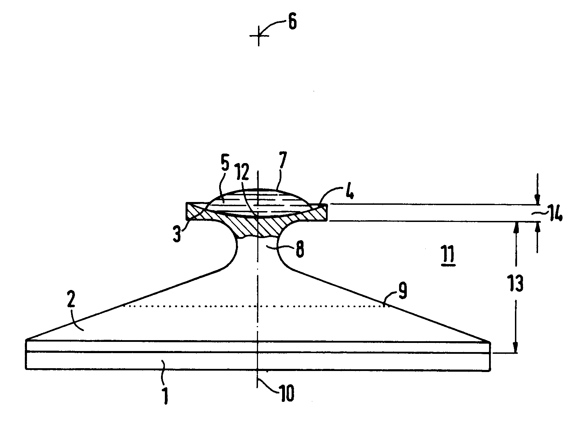

Die Erfindung wird anhand der Zeichnung näher erläutert, die einen Zerstäuber in Seitenansicht zeigt.

Bei dem Zerstäuber 11 ist auf eine Piezokeramikscheibe 1 ein Amplitudentransformator 2 aus CrNi-Stahl aufgeklebt. Mit 9 ist die neutrale Zone des Zerstäuberkegels bezeichnet, in der sich eine mechanische Dämpfung nicht durch eine Impedanzänderung bemerkbar macht. Auf dem oberen, sich zu einem Hals 8 verjüngenden Teil des Amplitudentransformators 2 ist eine hohlspiegelartige Verbreiterung 3 aus V2A-Stahl angebracht. In dem Hohlraum 4 befindet sich die zu zerstäubende Flüssigkeit 5, beispielsweise Bronchospasmalytika. Mit 7 ist die Oberfläche der zu zerstäubenden Flüssigkeit, mit 10 die Symmetrieachse, mit 12 der Schnittpunkt der Symmetrieachse mit der konkav gewölbten Oberfläche der plattenförmigen Verbreiterung 3, mit 6 Brennpunkt und mit 13 die Gesamthöhe des Amplitudentransformators gekennzeichnet.

Ein Ultraschallzerstäuber mit der erfindungsgemäßen Geometrie kann von der Piezokeramik 1 bis maximal zur Halshöhe 8 eingegossen werden. Ferner kann der Schwinger des Ultraschallzerstäubers problemlos unter einen O-Ring, der in der neutralen Zone 9 des Schwingers sitzt, eingespannt werden. Eingegossen in Silikonkautschuk zeigt der Schwinger gleich guten und effizienten Betrieb wie im nicht eingegossenen Zustand.

## Patentansprüche

1. Ultraschall-MHz-Schwinger mit einer Anregungsenergie $\leq$ 30 W (Watt) zur Zerstäubung von kleinsten Flüssigkeitsmengen zur Erzeugung eines lungengängigen Aerosols, mit einem sich im wesentlichen verjüngenden metallischen Amplitudentransformator (2), mit einer an seiner breiteren Basisfläche angekoppelten Piezokeramikscheibe (1) und mit einem an der hierzu gegenüberliegenden verjüngten Seite des Amplitudentransformators, die zu einem Hals (8) ausgebildet ist, befindlichen plattenförmigen Verbreiterung (3), die zur Halterung der Flüssigkeit bei Schräglagen mit durchgehend konkaver, hinsichtlich Durchmesser und Wölbung ganz an die zu zerstäubende Flüssigkeitsmenge und Flüssigkeitsoberflächenspannung angepaßter Oberfläche ohne Flüssigkeitszuführungsbohrung versehen ist und wobei die Höhe (13) des Amplitudentransformators zuzüglich der Dicke der plattenförmigen Verbreiterung (3) ein Vielfaches, vorzugsweise das Vier- bis Sechsfache, einer halben Wellenlänge des Ultraschalls im MHz-Bereich im Amplitudentransformator entspricht.

2. Ultraschall-MHz-Schwinger nach Anspruch 1, **dadurch gekennzeichnet,** daß der Abstand des Brennpunktes (6) der konkaven Verbreiterung (3) vom Schnittpunkt (12) der Symmetrieachse (10) mit der konkav gekrümmten Oberfläche der plattenförmigen Verbreiterung (3) dem zweifachen Durchmesser der Verbreiterung (3) entspricht.

3. Ultraschall-MHz-Schwinger nach Anspruch 1, **dadurch gekennzeichnet,** daß der Abstand des Brennpunktes (6) vom Schnittpunkt (12) der Symmetrieachse (10) mit der Verbreiterung (3) dem Durchmesser des Halses (8) entspricht.

4. Ultraschall-MHz-Schwinger nach Anspruch 1 und 2 oder 3, **dadurch gekennzeichnet,** daß der Durchmesser des Halses (8) auf die Arbeitsfrequenz des Zerstäubers abgestimmt ist.

5. Ultraschall-MHz-Schwinger nach Anspruch 4, **dadurch gekennzeichnet,** daß die Arbeitsfrequenz des Zerstäubers der Wellenlänge des Ultraschalls im Amplitudentransformator entspricht.

6. Ultraschall-MHz-Schwinger nach Anspruch 1 bis 5, **dadurch gekennzeichnet,** daß die Dikke (14) der Verbreiterung (3) einer halben Wellenlänge ($\lambda/2$) des Ultraschalls im Amplitudentransformator (2) entspricht.

7. Ultraschall-MHz-Schwinger nach Anspruch 1 bis 6, **dadurch gekennzeichnet,** daß die Höhe (13) des Amplitudentransformators (2) das Vier- bis Sechsfache der halben Wellenlänge im Amplitudentransformator (2) ist.

8. Ultraschall-MHz-Schwinger nach Anspruch 1 bis 7, **dadurch gekennzeichnet,** daß der Durchmesser der Verbreiterung (3) das 3,5-fache des Durchmessers vom Hals (8) beträgt.

9. Ultraschall-MHz-Schwinger nach Anspruch 1 bis 8, **dadurch gekennzeichnet,** daß die Dikke der Piezokeramikscheibe (1) der halben Wellenlänge in dieser Piezokeramikscheibe (1) entspricht.

10. Ultraschall-MHz-Schwinger nach Anspruch 1 bis 9, **dadurch gekennzeichnet,** daß die Gesamthöhe (13) des Ultraschallzerstäubers (11) das Vier- bis Sechsfache der halben Wellenlänge im Amplitudentransformator (2) beträgt und die Gesamthöhe (13) des Amplitudentransformators (2) sich zum Durchmesser der Piezokeramikscheibe (1) wie 1 : 2 verhält.

**Claims**

1. Ultrasonic MHz oscillator with an excitation power ≤ 30 W (Watt) for the atomization of the smallest quantities of liquid for the production of an aerosol inhaler, having a substantially tapering metallic amplitude transformer (2), with a piezoceramic disc (1) coupled to its wider base area and with a plate-shaped widening (3), located at the tapered side of the amplitude transformer, which side lies opposite the piezoceramic disc and is constructed to form a neck (8), which widening, for holding the liquid in sloping positions, is provided with a continuous concave surface, adapted with regard to its diameter and curvature entirely to the quantity of liquid to be atomized and to the surface tension of the liquid, without a borehole for the supply of the liquid, and whereby the height (13) of the amplitude transformer plus the thickness of the plate-shaped widening (3) corresponds to a multiple of, preferably four to six times, half a wavelength of the ultrasound in the MHz range in the amplitude transformer.

2. Ultrasonic MHz oscillator according to claim 1, characterized in that the distance of the focal point (6) of the concave widening (3) from the intersection (12) of the symmetry axis (10) with the concavely curved surface of the plate-shaped widening (3) corresponds to twice the diameter of the widening (3).

3. Ultrasonic MHz oscillator according to claim 1, characterized in that the distance of the focal point (6) from the intersection (12) of the symmetry axis (10) with the widening (3) corresponds to the diameter of the neck (8).

4. Ultrasonic MHz oscillator according to claim 1 and 2 or 3, characterized in that the diameter of the neck (8) is tuned to the operating frequency of the atomizer.

5. Ultrasonic MHz oscillator according to claim 4, characterized in that the operating frequency of the atomizer corresponds to the wavelength of the ultrasound in the amplitude transformer.

6. Ultrasonic Mhz oscillator according to claim 1 to 5, characterized in that the thickness (14) of the widening (3) corresponds to half a wavelength ($\lambda/_2$) of the ultrasound in the amplitude transformer (2).

7. Ultrasonic MHz oscillator according to claim 1 to 6, characterized in that the height (13) of the amplitude transformer (2) is four to six times half the wavelength in the amplitude transformer (2).

8. Ultrasonic MHz oscillator according to claim 1 to 7, characterized in that the diameter of the widening (3) amounts to 3.5 times the diameter of the neck (8).

9. Ultrasonic MHz oscillator according to claim 1 to 8, characterized in that the thickness of the piezoceramic disc (1) corresponds to half the wavelength in this piezoceramic disc (1).

10. Ultrasonic MHz oscillator according to claim 1 to 9, characterized in that the total height (13) of the ultrasonic atomizer (11) amounts to four to six times half the wavelength in the amplitude transformer (2) and the total height (13) of the amplitude transformer (2) is to the diameter of the piezoceramic disc (1) as 1 : 2.

**Revendications**

1. Vibrateur à ultrasons fonctionnant dans la gamme des MHz, alimenté par une énergie d'excitation ≤ 30 W (watts) pour pulvériser de très petites quantités de liquide de manière à produire un aérosol circulant dans les poumons, un transformateur métallique d'amplitude (2), possédant une forme sensiblement rétrécie, un disque piézocéramique (1) couplé à la surface de base plus large du transformateur et une partie élargie en forme de plaque (3), qui est située sur le côté rétréci, situé à l'opposé du disque, du transformateur d'amplitude et est agencé de manière à former un col (8) et qui, pour le support du liquide dans le cas de positions obliques, est pourvu d'une surface continûment concave, dont le diamètre et le cintrage sont entièrement adaptés à la quantité de liquide à pulvériser et à la tension superficielle de ce liquide; et ne comporte aucun perçage d'amenée de liquide, et dans lequel la hauteur (13) du transformateur d'amplitude, additionnée de l'épaisseur de l'élargissement en forme de plaque (3), correspond à un multiple, de préférence compris entre le quadruple et le sextuple, de la moitié de la longueur d'onde des ultrasons dans la gamme des MHz dans le transformateur d'amplitude.

2. Oscillateur à ultrasons fonctionnant dans la gamme des MHz suivant la revendication 1, caractérisé par le fait que la distance entre le foyer (6) de l'élargissement concave (3) et le point d'intersection (12) de l'axe de symétrie (10) et de la surface cintrée concave de l'élargissement en forme de plaque (3) correspond au double du diamètre de l'élargissement (3).

3. Oscillateur à ultrasons fonctionnant dans la gamme des MHz suivant la revendication 1, caractérisé par le fait que la distance entre le foyer (6) et le point d'intersection (12) de l'axe de symétrie (10) et de l'élargissement (3) correspond au diamètre du col (8).

4. Oscillateur à ultrasons dans la plage des MHz suivant les revendications 1 et 2 ou 3, caractérisé par le fait que le diamètre du col (8) est réglé sur la fréquence de travail du dispositif de pulvérisation.

5. Oscillateur à ultrasons fonctionnant dans la gamme des MHz suivant la revendication 4, caractérisé par le fait que la fréquence de travail du dispositif de pulvérisation correspond à la longueur d'onde des ultrasons dans le transformateur d'amplitude.

6. Oscillateur à ultrasons fonctionnant dans la plage des MHz suivant les revendications 1 à 5, caractérisé par le fait que l'épaisseur (14) de l'élargissement (3) correspond à la moitié ($\lambda/2$) de la longueur d'onde des ultrasons dans le transformateur d'amplitude (2).

7. Oscillateur à ultrasons fonctionnant dans la plage des MHz suivant les revendications 1 à 6, caractérisé par le fait que la hauteur (13) du transformateur d'amplitude (2) est comprise entre le quadruple et le sextuple de la moitié de la longueur d'onde dans le transformateur d'amplitude (2).

8. Oscillateur à ultrasons fonctionnant dans la plage des MHz suivant les revendications 1 à 7, caractérisé par le fait que le diamètre de l'élargissement (3) est égal à 3,5 fois le diamètre du col (8).

9. Oscillateur à ultrasons fonctionnant dans la plage des MHz suivant les revendications 1 à 8, caractérisé par le fait que l'épaisseur du disque piézocéramique (1) correspond à la moitié de la longueur d'onde dans ce disque piézocéramique (1).

10. Oscillateur à ultrasons fonctionnant dans la plage des MHz suivant les revendications 1 à 9, caractérisé par le fait que la hauteur totale (13) du dispositif de pulvérisation à ultrasons (11) est comprise entre le quadruple et le sextuple de la moitié de la longueur d'onde dans le transformateur d'amplitude (2) et que le rapport de la hauteur totale (13) du transformateur d'amplitude (2) au diamètre du disque piézocéramique (1) est égal à 1:2.